# EUROPÄISCHE PATENTSCHRIFT

(11) **EP 1 174 109 B1**
(45) Veröffentlichungstag und Bekanntmachung des Hinweises auf die Patenterteilung: **24.10.2007**
(21) Anmeldenummer: 01116613.9
(22) Anmeldetag: 12.07.2001
(51) Int. Cl.: A61K 8/06, A61K 8/34, A61K 8/73, A61Q 19/00, A61Q 1/02

(54) **Kosmetische Zubereitungen enthaltend Addukte von Cyclodextrinen und Coenzym Q 10**
Cosmetic preparations containing adducts of cyclodextrins and coenzyme Q 10
Préparations cosmétiques contenant des produits d'addition des cyclodextrines et de coenzyme Q 10

(30) Priorität: 21.07.2000 DE 10035513
(43) Veröffentlichungstag der Anmeldung: 23.01.2002
(73) Patentinhaber: Beiersdorf Aktiengesellschaft, 20245 Hamburg (DE)
(72) Erfinder: Nielsen, Jens, 24558 Henstedt-Ulzburg (DE); Max, Heiner, Dr., 22529 Hamburg (DE); Hargens, Birgit, 20257 Hamburg (DE); Schimpf, Ralph, Dr., 25474 Bönningstedt (DE)

(56) Entgegenhaltungen:
- EP-A- 0 233 615
- EP-A- 0 624 599
- WO-A-91/18589
- DE-A- 19 537 027
- US-A- 5 631 244
- DATABASE WPI Section Ch, Week 198141 Derwent Publications Ltd., London, GB; Class B04, AN 1981-74927D XP002260641 & JP 56 109590 A (ZERIA SHINYAKU KOGYO KK) , 31. August 1981 (1981-08-31)
- DATABASE WPI Section Ch, Week 198417 Derwent Publications Ltd., London, GB; Class B04, AN 1984-104277 XP002260642 & JP 59 047202 A (ZERIA SHINYAKU KOGYO KK) , 16. März 1984 (1984-03-16)
- DATABASE WPI Section Ch, Week 198403 Derwent Publications Ltd., London, GB; Class A96, AN 1984-014258 XP002260643 & JP 58 206540 A (FUJISAWA PHARM CO LTD), 1. Dezember 1983 (1983-12-01)
- DATABASE WPI Section Ch, Week 198821 Derwent Publications Ltd., London, GB; Class A96, AN 1988-142663 XP002260644 & JP 63 083021 A (KAO CORP), 13. April 1988 (1988-04-13)

## Beschreibung

Die vorliegende Erfindung betrifft kosmetische bzw. dermatologische dermatologische Zubereitungen, enthaltend Wirkstoffe zur Pflege und zum Schutze der Haut, insbesondere der empfindlichen und der trockenen Haut wie auch ganz besonders im Vordergrunde stehend der durch intrinsische und/oder extrinsische Faktoren gealterten oder alternden Haut sowie zur Unterstützung des hauteigenen Lipidmetabolismus sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säurehaltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Es werden ferner Antioxidantien, bevorzugt solche, welche in hautpflegenden kosmetischen oder dermatologischen Zubereitungen eingesetzt werden, beschrieben sowie kosmetische und dermatologische Zubereitungen, solche Antioxidantien enthaltend. Es werden kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung, beschrieben.

Weiterhin betrifft die vorliegende Erfindung Zubereitungen, die Wirkstoffe zur kosmetischen und dermatologischen Behandlung oder Prophylaxe erythematöser, entzündlicher, allergischer oder autoimmunreaktiver Erscheinungen, insbesondere Dermatosen, enthalten.

Die Zubereitungen dienen zur Prophylaxe und Behandlung der lichtempfindlichen Haut, insbesondere von Photodermatosen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Diesen Übelständen abzuhelfen, war Aufgabe der vorliegenden Erfindung.

Weiterhin sind kosmetische Zubereitungen mit Coenzym Q-10 aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

Coenzym Q-10 ist durch folgende Strukturformel gekennzeichnet

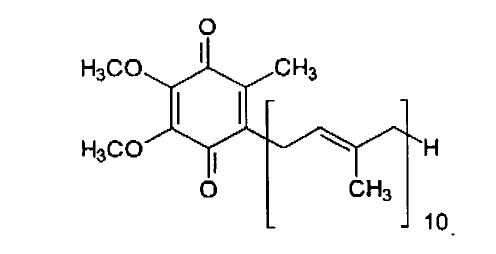

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q10 hat im Resultat eine wichtige Funktion bei der Prävention von Hautschäden durch UV-Strahlen und der Prävention von Hautalterung. Bei 20 bis 40-jährigen wird die Hautrauhigkeit gebessert, indem der Haut Feuchtigkeit gegeben wird.

Es war indes überraschend und für den Fachmann nicht vorauszusehen, daß kosmetische Zubereitungen in Form von Emulsionen vom Typ Wasser-in-ÖI (W/O) oder vom Typ ÖI-in-Wasser (O/W), oder multiplen Emulsionen, beispielsweise vom Typ Wasser-in-ÖI-in-Wasser (W/O/W) oder ÖI-in-Wasser-in-ÖI (O/W/O), von Hydrodispersionen oder Lipodispersionen, Gelen oder festen Stiften enthaltend molekulare Addukte von Cyclodextrinen und Coenzym Q10 den Nachteilen des Standes der Technik abhelfen,

Ubichinone stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q-10.

Ubichinone dienen den Organismen als Elektronenüberträger in der Atmungskette. Sie befinden sich in den Mitochondrien wo sie die cyclische Oxidation und Reduktion der Substrate des Citronensäure-Cyclus ermöglichen.

Cyclodextrine (Cycloamylosen, Cycloglucane) sind in kosmetischen und pharmazeutischen Zubereitungen an sich bekannt. Oftmals werden diese Substanzen zur "molekularen Verkapselung" verwendet, also als schützende Umhüllung empfindlicher Moleküle. Es handelt sich dabei um 6, 7, 8 oder noch mehr α-1,4-verknüpfte Glucoseeinheiten, wobei die Cyclohexaamylose (α-Cyclodextrin) sich durch die Struktur 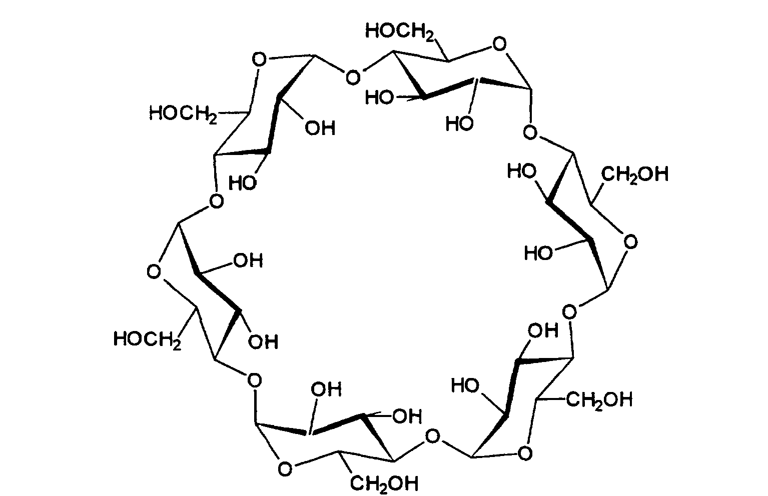 auszeichnet. Die Cycloheptaamylose (β-Cyclodextrin) zeichnet sich durch die Struktur 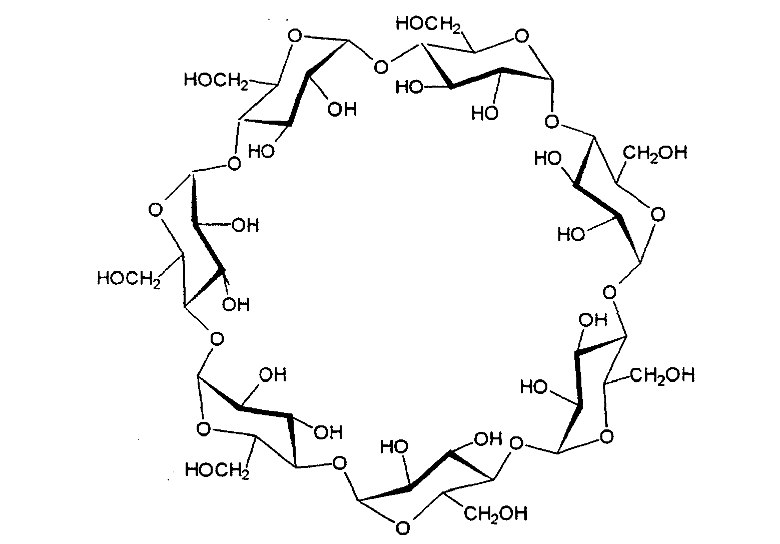 aus. Die Cyclooctaamylose (γ-Cyclodextrin) zeichnet sich durch die Struktur 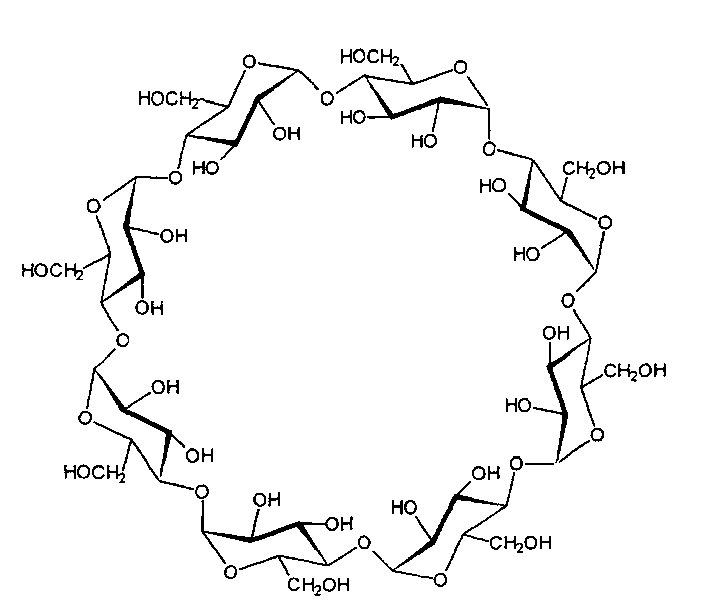 aus. Die Cycloenneaamylose (δ-Cyclodextrin) zeichnet sich durch die Struktur 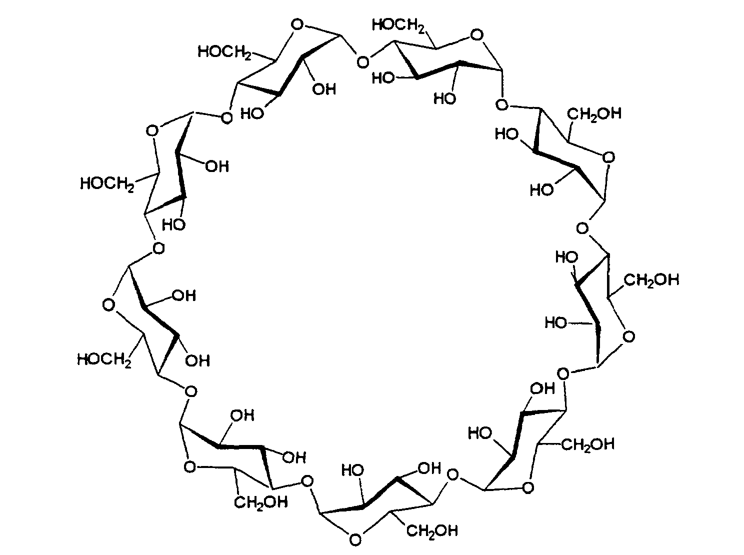 aus.

JP-A-56109590, JP-A-56047202, JP-A-58206540 offenbaren pharmazeutische Zubereitungen zur Behandlung innerer Erkrankungen, die Komplexe von Cyclodextrinen und Coenzym Q10 enthalten. EP-A-624599 und WO-A-91/18559 offenbaren kosmetische Zubereitungen in Form von Gelen oder Creme, die molekulare Addukte von Cyclodextrinen und lipophilen Aktivsubstanzen enthalten. Als lipophile Substanzen werden Hydrochinone genannt.

Weiterhin können im Rahmen dieses Patentes polar- und unpolar- substituierte Cyclodextrine eingesetzte werden. Hierzu gehören vorzugsweise aber nicht ausschließlich Methyl-, Ethyl- sowie Hydroxypropyl-Cyclodextrin.

Die beschriebenen Wirkstoffkombinationen lassen sich problemlos üblichen kosmetischen Zubereitungen, vorteilhaft Lichtschutzzubereitungen, aber auch gewünschtenfalls anderen Zubereitungen, beispielsweise pharmazeutischen Zubereitungen einverleiben.

Bei Anwendung des beschriebenen Wirkstoffes bzw. kosmetischer oder topischer dermatologischer Zubereitungen mit einem wirksamen Gehalt an erfindungsgemäß verwendetem Wirkstoff ist in überraschender Weise eine wirksame Behandlung, aber auch eine Prophylaxe
- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde
- von trockener Haut
- von lichtbedingten Hautschäden
- von Pigmentierungsstörungen,
- von Juckreiz,
- von trockenen Hautzuständen und Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo
möglich. Die kosmetischen oder topischen dermatologischen Zubereitungen mit einem wirksamen Gehalt an erfindungsgemäßem Wirkstoff dienen aber auch in überraschender Weise
- zur Beruhigung von empfindlicher oder gereizter Haut
- zur Stimulation der Kollagen-, Hyaluronsäure-, Elastinsynthese
- zur Stimulation der intrazellulären DNA-Synthese, insbesondere bei defizitären oder hypoaktiven Hautzuständen.
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Ferner war nicht vorauszusehen gewesen, daß das Coenzym Q10 in den erfindungsgemäßen Wirkstoffkombinationen höhere Stabilität gegenüber physikalischer oder chemischer Einwirkung, insbesondere Einwirkung von UV-Licht, aufweisen als wenn sie nicht mit Cyclodextrinen vergesellschaftet sind.

Es ist erfindungsgemäß insbesondere äußerst vorteilhaft, den erfindungsgemäß verwendeten Wirkstoff bzw. kosmetische oder topische dermatologische Zubereitungen mit einem wirksamen Gehalt an erfindungsgemäß verwendetem Wirkstoff zur kosmetischen oder dermatologischen Behandlung oder Prophylaxe unerwünschter Hautzustände zu verwenden.

Es wird vermutet, daß zumindest bei der vorab geschilderten direkten Vereinigung der Substanzen erfindungsgemäß molekulare Addukte aus Cyclodextrinen und Coenzym Q10 erhalten werden. Es wird daher im folgenden auch von erfindungsgemäßen molekularen Addukten die Rede sein, wenn erfindungsgemäße Wirkstoffkombinationen aus Cyclodextrinen und Coenzym Q10 besprochen werden.

Es bestehen gute Gründe, anzunehmen, daß solche molekularen Addukte in Analogie zu anderen molekularen Addukte der Cyclodextrine folgendem Schema folgen:

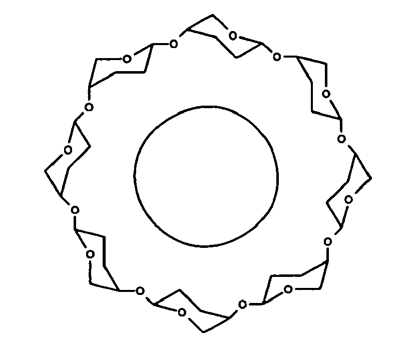

In diesem als wahrscheinlich anzunehmenden Schema stellen die Cyclodextringerüste das Wirtsmolekül und das betreffende Chinon bzw. Hxdrochinont, welche hier durch den Kreis im Innern des Schemas dargestellt sind, das Gastmolekül dar.

Aufgrund der errechneten molaren Verhältnisse sind erfindungsgemäß auch Wirkstoffkombinationen erhältlich, welche mit einiger Wahrscheinlichkeit als molekulare Addukte anzusehen sind, in welchen zwei, gegebenenfalls sogar noch mehr, identische oder unterschiedliche Gastmoleküle, welche hier durch Kreise im Innern des Schemas dargestellt sind, in einem Wirtsmolekül gleichsam auf molekularer Ebene verkapselt vorliegen. Dies wird im nachfolgenden Schema angedeutet. 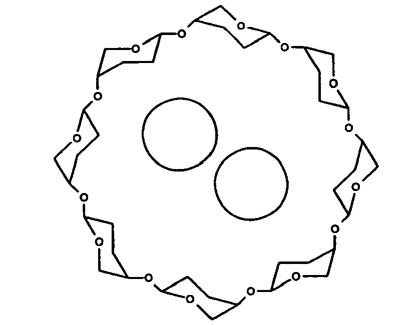 Solche molekularen Addukte bilden sich bevorzugt bei direkter Vereinigung der zugrundeliegenden Einzelsubstanzen, besonders bevorzugt, wenn die Vereinigung unter Vermittlung eines geeigneten Lösemittels erfolgt.

Vorteilhaft können erfindungsgemäße molekulare Addukte aus Cyclodextrinen und Wirkstoffkombinationen aus Cyclodextrinen und Coenzym Q10 beispielsweise erhalten werden, indem Cyclodextrine in Wasser gelöst werden und das Coenzym Q10 hinzugegeben werden. Das jeweilige molekulare Addukt fällt sodann als Festkörper aus und kann den üblichen Reinigungs- und Aufbereitungsschritten unterworfen werden.

Die Gesamtmenge an Coenzym Q10 in den fertigen kosmetischen oder dermatologischen Zubereitungen wird vorteilhaft aus dem Bereich von 0,001 - 10,0 Gew.-%, bevorzugt 0,01 - 5,0 Gew.-% gewählt, bezogen auf das Gesamtgewicht der Zubereitungen.

Die Gesamtmenge an Cyclodextrinen, insbesondere β-Cyclodextrin und/oder γ-Cyclodextrin in den fertigen kosmetischen oder dermatologischen Zubereitungen wird vorteilhaft aus dem Bereich von 0,001 - 20,0 Gew.-%, bevorzugt 0,02 - 10,0 Gew.-% gewählt, bezogen auf das Gesamtgewicht der Zubereitungen.

Besonders vorteilhaft ist es, Gewichtsverhältnisse von Cyclodextrinen zu Coenzym Q10 wie 10 : 1 bis 1 : 5, bevorzugt wie 8 : 1 bis 1 : 2, besonders bevorzugt wie 4 : 1 bis 1 : 1 zu wählen.

Insbesondere vorteilhaft sind als molekulare Addukte aus Cyclodextrinen und mindestens einem Chinon und/oder mindestens einem Hydrochinon angesehene Wirkstoffkombinationen welche die folgenden molaren Verhältnisse aufweisen:
1 mol α-Cyclodextrin : 1 mol Coenzym Q10
1 mol β-Cyclodextrin : 1 mol Coenzym Q10
1 mol γ-Cyclodextrin : 1 mol Coenzym Q10
2 mol α-Cyclodextrin : 1 mol Coenzym Q10
2 mol β-Cyclodextrin : 1 mol Coenzym Q10
2 mol γ-Cyclodextrin : 1 mol Coenzym Q10
1 mol α-Cyclodextrin : 2 mol Coenzym Q10
1 mol β-Cyclodextrin : 2 mol Coenzym Q10
1 mol γ-Cyclodextrin : 2 mol Coenzym Q10
3 mol α-Cyclodextrin : 1 mol Coenzym Q10
3 mol β-Cyclodextrin : 1 mol Coenzym Q10
3 mol γ-Cyclodextrin : 1 mol Coenzym Q10

Erfindungsgemäß bevorzugte Wirkstoffkombinationen betreffen Coenzym Q10 und γ-Cyclodextrin, insbesondere bevorzugt in molaren Verhältnissen von γ-Cyclodextrin zu Coenzym Q10 wie 1,5 zu 1 bis 2,5 zu 1, insbesondere etwa wie 2 zu 1.

Erfindungsgemäße kosmetische und dermatologische Zubereitungen enthalten außerdem vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃, Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Die anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

n TiO₂ + m (RO)₃Si-R' -> n TiO₂ (oberfl.)

erzeugt wird n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Der verwendete Wirkstoff wird eingearbeitet in übliche kosmetische und dermatologische Zubereitungen, welche in Form von Emulsionen vom Typ Wasser-in-Öl (W/O) oder vom Typ Öl-in-Wasser (O/W), oder von multiplen Emulsionen, beispielsweise vom Typ Wasser-in-Öl-in-Wasser (W/O/W) oder Öl-in-Wasser-in-Öl (O/W/O), von Hydrodispersionen oder Lipodispersionen, einem Gel oder einem festen Stift vorliegen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind gegebenenfalls auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäße, als Emulsionen vorliegende Zubereitungen enthalten einen oder mehrere Emulgatoren. O/W-EMulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel R-COO-(-CH₂-CH₂-O-)ₙ-R',
- der veresterten Fettsäureethoxylate der allgemeinen Formel R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H,
- der Polypropylenglycolether der allgemeinen Formel R-O-(-CH₂-CH(CH₃)-O-)ₙ-R',
- der propoxylierten Wollwachsalkohole,
- der veretherten Fettsäurepropoxylate R-COO-(-CH₂-CH(CH₃)-O-)ₙ-R',
- der veresterten Fettsäurepropoxylate der allgemeinen Formel R-COO-(-CH₂-CH(CH₃)-O-)ₙ-C(O)-R',
- der Fettsäurepropoxylate der allgemeinen Formel R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel R-O-Xₙ-Yₘ-H,
- der Polypropylenglycolether der allgemeinen Formel R-O-Xₙ-Yₘ-R',
- der veretherten Fettsäurepropoxylate der allgemeinen Formel R-COO-Xₙ-Yₘ-R',
- der Fettsäureethoxylate/propoxylate der allgemeinen Formel R-COO-Xₙ-Yₘ-H,

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)-glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Als Emulgatoren eignen sich besonders Glycerylstearatcitrat, Glycerylstearat, PEG-Ester der Stearinsäure sowie die Kombination GlycerylstearatlStearinsäure.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Herstellungsbeispiel 1

In eine gesättigte, wäßrige α-Cyclodextrin-Lösung wird unter intensivem Rühren bei Raumtemperatur ein halbes Moläquivalent, bezogen auf die Cyclodextrinmenge, Coenzym Q10 langsam zugegeben. Es wird über Nacht weiter gerührt. Das ausgefallene Produkt (= molekulares Addukt 1) wird über Keramikfilter unter Vakuum filtriert, anschließend mit destilliertem Wasser gewaschen, getrocknet und zu mikronisierten Partikeln gemahlen.

### Herstellungsbeispiel 2

In eine gesättigte, wäßrige α-Cyclodextrin-Lösung wird unter intensivem Rühren bei 50°C ein halbes Moläquivalent, bezogen auf die Cyclodextrinmenge, von Coenzym Q10 in Form einer wässrigen Suspension langsam zugegeben. Man läßt auf Raumtemperatur abkühlen und über Nacht weiterrühren. Das ausgefallene Produkt (= molekulares Addukt 2) wird über Keramikfilter unter Vakuum filtriert, anschließend mit destilliertem Wasser gewaschen, getrocknet und zu mikronisierten Partikeln gemahlen.

### Herstellungsbeispiel 3

In eine gesättigte, wäßrige β-Cyclodextrin-Lösung wird unter intensivem Rühren bei Raumtemperatur ein halbes Moläquivalent, bezogen auf die Cyclodextrinmenge, Coenzym Q10 langsam zugegeben. Es wird über Nacht weiter gerührt. Das ausgefallene Produkt (= molekulares Addukt 3) wird über Keramikfilter unter Vakuum filtriert, anschließend mit destilliertem Wasser gewaschen, getrocknet und zu mikronisierten Partikeln gemahlen.

### Herstellungsbeispiel 4

In eine gesättigte, wäßrige β-Cyclodextrin-Lösung wird unter intensivem Rühren bei 70°C ein halbes Moläquivalent, bezogen auf die Cyclodextrinmenge, von Coenzym Q10 in Form einer wässrigen Suspension langsam zugegeben. Man läßt auf Raumtemperatur abkühlen und über Nacht weiterrühren. Das ausgefallene Produkt (= molekulares Addukt 4) wird über Keramikfilter unter Vakuum filtriert, anschließend mit destilliertem Wasser gewaschen, getrocknet und zu mikronisierten Partikeln gemahlen.

### Herstellungsbeispiel 5

In eine gesättigte, wäßrige γ-Cyclodextrin-Lösung wird unter intensivem Rühren bei Raumtemperatur ein halbes Moläquivalent, bezogen auf die Cyclodextrinmenge, Coenzym Q10 langsam zugegeben. Es wird über Nacht weiter gerührt. Das ausgefallene Produkt (= molekulares Addukt 5) wird über Keramikfilter unter Vakuum filtriert, anschließend mit destilliertem Wasser gewaschen, getrocknet und zu mikronisierten Partikeln gemahlen.

### Herstellungsbeispiel 6

In eine gesättigte, wäßrige γ-Cyclodextrin-Lösung wird unter intensivem Rühren bei 80°C ein halbes Moläquivalent, bezogen auf die Cyclodextrinmenge, von Coenzym Q10 in Form einer wässrigen Suspension langsam zugegeben. Man läßt auf Raumtemperatur abkühlen und über Nacht weiterrühren. Das ausgefallene Produkt (= molekulares Addukt 6) wird über Keramikfilter unter Vakuum filtriert, anschließend mit destilliertem Wasser gewaschen, getrocknet und zu mikronisierten Partikeln gemahlen.

### Beispiel 1 (O/W-Creme):

| | Gew.-% |
|---|---|
| Glycerylstearatcitrat | 2,00 |
| Stearylalkohol | 5,00 |
| Caprylisäure/Caprinsäuretriglyceride | 4,00 |
| Octyldodecanol | 4,00 |
| Glycerin | 3,00 |
| Carbomer | 0,10 |
| molekulares Addukt 1 | |
| (Coenzym Q10, aktiv in α-Cyclodextrin-Komplex) | 0,015 |
| Natriumhydroxid | q.s. |
| Konservierung | q.s. |
| Parfum | q.s. |
| Wasser,demineralisiert | ad 100,00 |
| pH-Wert eingestellt auf 6.0 | |

### Beispiel 2 (O/W-Creme):

| | Gew.-% |
|---|---|
| Glycerylstearatcitrat | 3,00 |
| Cetylstearylalkohol | 3,00 |
| Paraffin Oil | 2,00 |
| Caprylsäure-/Caprinsäuretriglyceride | 4,00 |
| Dicaprylylether | 3,00 |
| Xanthangummi | 0,10 |
| Citronensäure | 0,10 |
| Natriumcitrat | 0,20 |
| molekulares Addukt 1 | |
| (Coenzym Q10, aktiv in α-Cyclodextrin-Komplex) | 0,010 |
| Glycerin | 3,00 |
| Konservierung | q.s. |
| Parfum | q.s. |
| Wasser | ad 100,00 |
| pH-Wert eingestellt auf 5.5 | |

### Beispiel 3 (O/W-Creme):

| | Gew.-% |
|---|---|
| Glycerylstearat SE | 4,00 |
| PEG-40 Stearate | 1,00 |
| Cetylalkohol | 3,00 |
| Caprylsäure-lCaprinsäuretriglyceride | 5,00 |
| Paraffin Oil | 5,00 |
| Glycerin | 3,00 |
| Carbomer | 0,10 |
| molekulares Addukt 1 | |
| (Coenzym Q10, aktiv in α-Cyclodextrin-Komplex) | 0,025 |
| Natriumhydroxid | q.s. |
| Konservierung | q.s. |
| Parfum | q.s. |
| Wasser,demineralisiert | ad 100,00 |
| pH-Wert eingestellt auf 5.0 | |

### Beispiel 4 (O/W-Creme):

| | Gew.-% |
|---|---|
| Glycerylstearat SE | 3,00 |
| Stearinsäure | 1,00 |
| Cetylalkohol | 2,00 |
| Dicaprylylether | 4,00 |
| Caprylsäure-/Caprinsäuretriglyceride | 3,00 |
| Paraffin Oil | 2,00 |
| Glycerin | 3,00 |
| Butylenglycol | 3,00 |
| Carbomer | 0,10 |
| molekulares Addukt 1 | |
| (Coenzym Q10, aktiv in α-Cyclodextrin-Komplex) | 0,020 |
| Natriumhydroxid | q.s. |
| Konservierung | q.s. |
| Parfum | q.s. |
| Wasser,demineralisiert | ad 100,00 |
| pH-Wert eingestellt auf 7.0 | |

### Beispiel 5 (O/W-Lotion):

| | Gew.-% |
|---|---|
| Glycerylstearat, Ceteth-20 | 1,00 |
| Sorbitan Stearate | 1,00 |
| Stearylalkohol | 1,00 |
| Caprylsäure-/Caprinsäuretriglyceride | 2,00 |
| Paraffinöl | 4,00 |
| Glycerin | 3,00 |
| Carbomer | 0,10 |
| molekulares Addukt 1 | |
| (Coenzym Q10, aktiv in α-Cyclodextrin-Komplex) | 0,010 |
| Natriumhydroxid | q.s. |
| Konservierung | q.s. |
| Parfum | q.s. |
| Wasser,demineralisiert | ad 100,00 |
| pH-Wert eingestellt auf 5.5 | |

### Beispiel 6 (Emulsions-Make-up):

| | Gew.-% |
|---|---|
| Glycerylstearat SE | 5,00 |
| Stearylalkohol | 2,00 |
| Dimethicon | 2,00 |
| Glycerin | 3,00 |
| Carbomer | 0,15 |
| Glimmer | 1,00 |
| Magnesiumsilikat | 1,00 |
| Eisenoxide | 1,00 |
| Titandioxid | 2,50 |
| Talkum | 5,00 |
| molekulares Addukt 1 | |
| (Coenzym Q10, aktiv in α-Cyclodextrin-Komplex) | 0,015 |
| Natriumhydroxid | q.s. |
| Konservierung | q.s. |
| Parfum | q.s. |
| Wasser,demineralisiert | ad 100,00 |
| pH-Wert eingestellt auf 6.0 | |

### Beispiel 7 (W/O/W-Creme):

| | Gew.-% |
|---|---|
| Glycerylstearat SE | 3,00 |
| PEG-100-stearat | 0,75 |
| Behenylalkohol | 2,00 |
| Caprylsäure-/Caprinsäuretriglyceride | 8,0 |
| Octyldodecanol | 5,00 |
| C₁₂₋₁₅ Alkylbenzoate | 3,00 |
| Panthenol | 3,00 |
| MgSO₄ | 0,80 |
| molekulares Addukt 1 | |
| (Coenzym Q10, aktiv in α-Cyclodextrin-Komplex) | 0,020 |
| Konservierung | q.s. |
| Parfum | q.s. |
| Wasser,demineralisiert | ad 100,00 |
| pH-Wert eingestellt auf 6.0 | |

### Beispiel 8 (hydrodispersionsgel):

| | Gew.-% |
|---|---|
| Carbomer | 0,40 |
| Xanthangummi | 0,20 |
| Cetylstearylalkohol | 2,00 |
| C₁₂₋₁₅ Alkylbenzoate | 5,00 |
| Caprylsäure-/Caprinsäuretriglyceride | 3,00 |
| Glycerin | 3,00 |
| molekulares Addukt 1 | |
| (Coenzym Q10, aktiv in α-Cyclodextrin-Komplex) | 0,020 |
| Natriumhydroxid | q.s. |
| Konservierung | q.s. |
| Parfum | q.s. |
| Wasser,demineralisiert | ad 100,00 |
| pH-Wert eingestellt auf 5.5 | |

### Beispiel 9 (W/O-Creme):

| | Gew.-% |
|---|---|
| PEG-7 Hydriertes Ricinusöl | 4,00 |
| Wollwachsalkohol | 1,50 |
| Bienenwachs | 3,00 |
| Paraffinöl | 10,00 |
| Caprylsäure-/Caprinsäuretriglyceride | 5,00 |
| Vaseline | 7,00 |
| Glycerin | 3,00 |
| MgS04 | 0,70 |
| molekulares Addukt 2 | |
| (Coenzym Q10, aktiv in α-Cyclodextrin-Komplex) | 0,010 |
| Konservierung | q.s. |
| Parfum | q.s. |
| Wasser,demineralisiert | ad 100,00 |

### Beispiel 10 O/W-Creme):

| | Gew.-% |
|---|---|
| Glycerylstearatcitrat | 2,00 |
| Stearylalkohol | 5,00 |
| Caprylisäure/Caprinsäuretriglyceride | 4,00 |
| Octyldodecanol | 4,00 |
| Glycerin | 3,00 |
| Carbomer | 0,10 |
| molekulares Addukt 3 | |
| (Coenzym Q10, aktiv in β-Cyclodextrin-Komplex) | 0,015 |
| Natriumhydroxid | q.s. |
| Konservierung | q.s. |
| Parfum | q.s. |
| Wasser,demineralisiert | ad 100,00 |
| pH-Wert eingestellt auf 6.0 | |

### Beispiel 11 (O/W-Creme):

| | Gew.-% |
|---|---|
| Glycerylstearatcitrat | 3,00 |
| Cetylstearylalkohol | 3,00 |
| Paraffin Oil | 2,00 |
| Caprylsäure-/Caprinsäuretriglyceride | 4,00 |
| Dicaprylylether | 3,00 |
| Xanthangummi | 0,10 |
| Citronensäure | 0,10 |
| Natriumcitrat | 0,20 |
| molekulares Addukt 3 | |
| (Coenzym Q10, aktiv in β-Cyclodextrin-Komplex) | 0,010 |
| Glycerin | 3,00 |
| Konservierung | q. s. |
| Parfum | q.s. |
| Wasser | ad 100,00 |
| pH-Wert eingestellt auf 5.5 | |

### Beispiel 12 (O/W-Creme):

| | Gew.-% |
|---|---|
| Glycerylstearat SE | 4,00 |
| PEG-40 Stearate | 1,00 |
| Cetylalkohol | 3,00 |
| Caprylsäure-/Caprinsäuretriglyceride | 5,00 |
| Paraffin Oil | 5,00 |
| Glycerin | 3,00 |
| Carbomer | 0,10 |
| molekulares Addukt 3 | |
| (Coenzym Q10, aktiv in β-Cyclodextrin-Komplex) | 0,025 |
| Natriumhydroxid | q.s. |
| Konservierung | q.s. |
| Parfum | q.s. |
| Wasser,demineralisiert | ad 100,00 |
| pH-Wert eingestellt auf 5.0 | |

### Beispiel 13 (O/W-Creme):

| | Gew.-% |
|---|---|
| Glycerylstearat SE | 3,00 |
| Stearinsäure | 1,00 |
| Cetylalkohol | 2,00 |
| Dicaprylylether | 4,00 |
| Caprylsäure-/Caprinsäuretriglyceride | 3,00 |
| Paraffin Oil | 2,00 |
| Glycerin | 3,00 |
| Butylenglycol | 3,00 |
| Carbomer | 0,10 |
| molekulares Addukt 3 | |
| (Coenzym Q10, aktiv in β-Cyclodextrin-Komplex) | 0,020 |
| Natriumhydroxid | q.s. |
| Konservierung | q.s. |
| Parfum | q.s. |
| Wasser,demineralisiert | ad 100,00 |
| pH-Wert eingestellt auf 7.0 | |

### Beispiel 14 (O/W-Lotion):

| | Gew.-% |
|---|---|
| Glycerylstearat, Ceteth-20 | 1,00 |
| Sorbitan Stearate | 1,00 |
| Stearylalkohol | 1,00 |
| Caprylsäure-/Capnnsäuretriglyceride | 2,00 |
| Paraffinöl | 4,00 |
| Glycerin | 3,00 |
| Carbomer | 0,10 |
| molekulares Addukt 3 | |
| (Coenzym Q10, aktiv in β-Cyclodextrin-Komplex) | 0,010 |
| Natriumhydroxid | q.s. |
| Konservierung | q.s. |
| Parfum | q.s. |
| Wasser,demineralisiert | ad 100,00 |
| pH-Wert eingestellt auf 5.5 | |

### Beispiel 15 (Emulsions-Make-up):

| | Gew.-% |
|---|---|
| Glycerylstearat SE | 5,00 |
| Stearylalkohol | 2,00 |
| Dimethicon | 2,00 |
| Glycerin | 3,00 |
| Carbomer | 0,15 |
| Glimmer | 1,00 |
| Magnesiumsilikat | 1,00 |
| Eisenoxide | 1,00 |
| Titandioxid | 2,50 |
| Talkum | 5,00 |
| molekulares Addukt 3 | |
| (Coenzym Q10, aktiv in β-Cyclodextrin-Komplex) | 0,015 |
| Natriumhydroxid | q.s. |
| Konservierung | q.s. |
| Parfum | q.s. |
| Wasser,demineralisiert | ad 100,00 |
| pH-Wert eingestellt auf 6.0 | |

### Beispiel 16 (W/O/W-Creme):

| | Gew.-% |
|---|---|
| Glycerylstearat SE | 3,00 |
| PEG-100-stearat | 0,75 |
| Behenylalkohol | 2,00 |
| Caprylsäure-/Caprinsäuretriglyceride | 8,0 |
| Octyldodecanol | 5,00 |
| C₁₂₋₁₅ Alkylbenzoate | 3,00 |
| Panthenol | 3,00 |
| MgSO₄ | 0,80 |
| molekulares Addukt 3 | |
| (Coenzym Q10, aktiv in β-Cyclodextrin-Komplex) | 0,020 |
| Konservierung | q.s. |
| Parfum | q.s. |
| Wasser,demineralisiert | ad 100,00 |
| pH-Wert eingestellt auf 6.0 | |

### Beispiel 17 (Hydrodispersionsgel):

| | Gew.-% |
|---|---|
| Carbomer | 0,40 |
| Xanthangummi | 0,20 |
| Cetylstearylalkohol | 2,00 |
| C₁₂₋₁₅ Alkylbenzoate | 5,00 |
| Caprylsäure-/Caprinsäuretriglyceride | 3,00 |
| Glycerin | 3,00 |
| molekulares Addukt 1 | |
| (Coenzym Q10, aktiv in β-Cyclodextrin-Komplex) | 0,020 |
| Natriumhydroxid | q.s. |
| Konservierung | q.s. |
| Parfum | q.s. |
| Wasser,demineralisiert | ad 100,00 |
| pH-Wert eingestellt auf 5.5 | |

### Beispiel 18 (W/O-Creme):

| | Gew.-% |
|---|---|
| PEG-7 Hydriertes Ricinusöl | 4,00 |
| Wollwachsalkohol | 1,50 |
| Bienenwachs | 3,00 |
| Paraffinöl | 10,00 |
| Caprylsäure-/Caprinsäuretriglyceride | 5,00 |
| Vaseline | 7,00 |
| Glycerin | 3,00 |
| MgSO4 | 0,70 |
| molekulares Addukt 4 | |
| (Coenzym Q10, aktiv in β-Cyclodextrin-Komplex) | 0,010 |
| Konservierung | q.s. |
| Parfum | q.s. |
| Wasser,demineralisiert | ad 100,00 |

### Beispiel 19 (O/W-Creme):

| | Gew.-% |
|---|---|
| Glycerylstearatcitrat | 2,00 |
| Stearylalkohol | 5,00 |
| Caprylisäure/Caprinsäuretriglyceride | 4,00 |
| Octyldodecanol | 4,00 |
| Glycerin | 3,00 |
| Carbomer | 0,10 |
| molekulares Addukt 5 | |
| (Coenzym Q10, aktiv in γ-Cyclodextrin-Komplex) | 0,015 |
| Natriumhydroxid | q.s. |
| Konservierung | q.s. |
| Parfum | q.s. |
| Wasser,demineralisiert | ad 100,00 |
| pH-Wert eingestellt auf 6.0 | |

### Beispiel 20 (O/W-Creme):

| | Gew.-% |
|---|---|
| Glycerylstearatcitrat | 3,00 |
| Cetylstearylalkohol | 3,00 |
| Paraffin Oil | 2,00 |
| Caprylsäure-/Caprinsäuretriglyceride | 4,00 |
| Dicaprylylether | 3,00 |
| Xanthangummi | 0,10 |
| Citronensäure | 0,10 |
| Natriumcitrat | 0,20 |
| molekulares Addukt 5 | |
| (Coenzym Q10, aktiv in γ-Cyclodextrin-Komplex) | 0,010 |
| Glycerin | 3,00 |
| Konservierung | q.s. |
| Parfum | q.s. |
| Wasser | ad 100,00 |
| pH-Wert eingestellt auf 5.5 | |

### Beispiel 21 (O/W-Creme):

| | Gew.-% |
|---|---|
| Glycerylstearat SE | 4,00 |
| PEG-40 Stearate | 1,00 |
| Cetylalkohol | 3,00 |
| Caprylsäure-/Caprinsäuretriglyceride | 5,00 |
| Paraffin Oil | 5,00 |
| Glycerin | 3,00 |
| Carbomer | 0,10 |
| molekulares Addukt 5 | |
| (Coenzym Q10, aktiv in γ-Cyclodextrin-Komplex) | 0,025 |
| Natriumhydroxid | q.s. |
| Konservierung | q.s. |
| Parfum | q.s. |
| Wasser,demineralisiert | ad 100,00 |
| pH-Wert eingestellt auf 5.0 | |

### Beispiel 22 (O/W-Creme):

| | Gew.-% |
|---|---|
| Glycerylstearat SE | 3,00 |
| Stearinsäure | 1,00 |
| Cetylalkohol | 2,00 |
| Dicaprylylether | 4,00 |
| Caprylsäure-/Caprinsäuretriglyceride | 3,00 |
| Paraffin Oil | 2,00 |
| Glycerin | 3,00 |
| Butylenglycol | 3,00 |
| Carbomer | 0,10 |
| molekulares Addukt 5 | |
| (Coenzym Q10, aktiv in γ-Cyclodextrin-Komplex) | 0,020 |
| Natriumhydroxid | q.s. |
| Konservierung | q.s. |
| Parfum | q.s. |
| Wasser,demineralisiert | ad 100,00 |
| pH-Wert eingestellt auf 7.0 | |

### Beispiel 23 (O/W-Lotion):

| | Gew.-% |
|---|---|
| Glycerylstearat , Ceteth-20 | 1,00 |
| Sorbitan Stearate | 1,00 |
| Stearylalkohol | 1,00 |
| Caprylsäure-/Caprinsäuretriglyceride | 2,00 |
| Paraffinöl | 4,00 |
| Glycerin | 3,00 |
| Carbomer | 0,10 |
| molekulares Addukt 5 | |
| (Coenzym Q10, aktiv in γ-Cyclodextrin-Komplex) | 0,010 |
| Natriumhydroxid | q.s. |
| Konservierung | q.s. |
| Parfum | q.s. |
| Wasser,demineralisiert | ad 100,00 |
| pH-Wert eingestellt auf 5.5 | |

### Beispiel 24 (Emulsions-Make-up):

| | Gew.-% |
|---|---|
| Glycerylstearat SE | 5,00 |
| Stearylalkohol | 2,00 |
| Dimethicon | 2,00 |
| Glycerin | 3,00 |
| Carbomer | 0,15 |
| Glimmer | 1,00 |
| Magnesiumsilikat | 1,00 |
| Eisenoxide | 1,00 |
| Titandioxid | 2,50 |
| Talkum | 5,00 |
| molekulares Addukt 5 | |
| (Coenzym Q10, aktiv in γ-Cyclodextrin-Komplex) | 0,015 |
| Natriumhydroxid | q.s. |
| Konservierung | q.s. |
| Parfum | q.s. |
| Wasser,demineralisiert | ad 100,00 |
| pH-Wert eingestellt auf 6.0 | |

### Beispiel 25 (W/O/W.Creme):

| | Gew.-% |
|---|---|
| Glycerylstearat SE | 3,00 |
| PEG-100-stearat | 0,75 |
| Behenylalkohol | 2,00 |
| Caprylsäure-/Caprinsäuretriglyceride | 8,0 |
| Octyldodecanol | 5,00 |
| C₁₂₋₁₅ Alkylbenzoate | 3,00 |
| Panthenol | 3,00 |
| MgSO₄ | 0,80 |
| molekulares Addukt 5 | |
| (Coenzym Q10, aktiv in γ-Cyclodextrin-Komplex) | 0,020 |
| Konservierung | q.s. |
| Parfum | q.s. |
| Wasser,demineralisiert | ad 100,00 |
| pH-Wert eingestellt auf 6.0 | |

### Beispiel 26 (Hydrodispersionsgel):

| | Gew.-% |
|---|---|
| Carbomer | 0,40 |
| Xanthangummi | 0,20 |
| Cetylstearylalkohol | 2,00 |
| C₁₂₋₁₅ Alkylbenzoate | 5,00 |
| Caprylsäure-/Caprinsäuretriglyceride | 3,00 |
| Glycerin | 3,00 |
| molekulares Addukt 5 | |
| (Coenzym Q10, aktiv in γ-Cyclodextrin-Komplex) | 0,020 |
| Natriumhydroxid | q.s. |
| Konservierung | q. s. |
| Parfum | q.s. |
| Wasser demineralisiert | ad 100,00 |
| pH-Wert eingestellt auf 5.5 | |

## Patentansprüche

1. Kosmetische Zubereitungen in Form von Emulsionen vom Typ Wasser-in-Öl (W/O) oder vom Typ Öl-in-Wasser (O/W), oder multiplen Emulsionen, beispielsweise vom Typ Wasser-in-Öl-in-Wasser (W/O/W) oder Öl-in-Wasser-in-Öl (O/W/O), von Hydrodispersionen oder Lipodispersionen, Gelen oder festen Stiften enthaltend molekulare Addukte von Cyclodextrinen und Coenzym Q10.

2. Zubereitungen nach Anspruch 1, **dadurch gekennzeichnet, daß** die Gesamtmenge an Coenzym Q10. in den fertigen kosmetischen oder dermatologischen Zubereitungen aus dem Bereich von 0,001 - 10,0 Gew.-%, bevorzugt 0,01 - 5,0 Gew.-% gewählt wird, bezogen auf das Gesamtgewicht der Zubereitungen.

3. Zubereitungen nach Anspruch 1, **dadurch gekennzeichnet, daß** die Gesamtmenge an Cyclodextrin in den fertigen kosmetischen oder dermatologischen Zubereitungen aus dem Bereich von 0,001 - 20,0 Gew.%, bevorzugt 0,02 - 10,0 Gew.-% gewählt wird, bezogen auf das Gesamtgewicht der Zubereitungen.

4. Zubereitungen nach Anspruch 1, **dadurch gekennzeichnet, daß** das oder die molekularen Addukte aus Cyclodextrinen und Coenzym Q10 die folgenden molaren Verhältnisse aufweisen:
1 mol α-Cytlodextrin : 1 mol Coenzym Q10
1 mol β-Cycladextrin : 1 mol Coenzym Q10
1 mol γ-Cyclodextrin : 1 mol Coenzym Q10
2 mol α-Cyclodextrin : 1 mol Coenzym Q10
2 mol β-Cyclodextrin : 1 mol Coenzym Q10
2 mol γ-Cyclodextrin : 1 mol Coenzym Q10
1 mol α-Cyclodextrin : 2 mol Coenzym Q10
1 mol β-Cyclodextrin : 2 mol Coenzym Q10
1 mol γ-Cyclodextrin : 2 mol Coenzym Q10
3 mol α-Cyclodextrin : 1 mol Coenzym Q10
3 mol β-Cyclodextrin : 1 mol Coenzym Q10
3 mol γ-Cyclodextrin : 1 mol Coenzym Q10.

## Claims

1. Cosmetic preparations in the form of emulsions of the type water-in-oil (W/O) or of the type oil-in-water (O/W), or multiple emulsions, for example of the type water-in-oil-in-water (W/O/W) or oil-in-water-in-oil (O/W/O), hydrodispersions or lipodispersions, gels or solid pens containing molecular adducts of cyclodextrins and coenzyme 010.

2. Preparations according to claim 1, wherein the total amount of coenzyme Q10 in the finished cosmetic or dermatological preparations is chosen from the range from 0.001 - 10.0 % by weight, preferably 0.01 - 5.0 % by weight, in relation to the total weight of the preparations.

3. Preparations according to claim 1, wherein the total amount of cyclodextrin in the finished cosmetic or dermatological preparations is chosen from the range from 0.001 - 20.0 % by weight, preferably 0.02 - 10.0 % by weight, in relation to the total weight of the preparations.

4. Preparations according to claim 1, wherein the molecular adduct or adducts from cyclodextrins and coenzyme 010 exhibit the following ratios:
1 mole α-cyclodextrin : 1 mole coenzyme Q10
1 mole β-cyclodextrin : 1 mole coenzyme Q10
1 mole γ-cyclodextrin : 1 mole coenzyme Q10
2 moles α-cyclodextrin : 1 mole coenzyme Q10
2 moles β-cyclodextrin : 1 mole coenzyme Q10
2 moles γ-cyclodextrin : 1 mole coenzyme Q10
1 mole α-cyclodextrin : 2 moles coenzyme Q10
1 mole β-cyclodextrin : 2 moles coenzyme Q10
1 mole γ-cyclodextrin : 2 moles coenzyme Q10
3 moles α-cyclodextrin : 1 mole coenzyme Q10
3 moles β-cyclodextrin : 1 mole coenzyme Q10
3 moles γ-cyclodextrin : 1 mole coenzyme Q10

## Revendications

1. Des préparations cosmétiques sous forme d'émulsions de type eau dans l'huile (E/H) ou du type huile dans l'eau (H/E) ou des émulsions muötiples par exemple du type eau dans huile dans eau (E/H/E) ou huile dans l'eau dans l'huile (H/E/H) d'hydrodispersions ou des lipodispersions, des gels ou des bâtons solides contenant des ajouts moléculaires de cyclodextrines et de coenzyme Q 10.

2. Des préparations selon la revendication 1 **caractérisées par le fait que** la quantité totale de coenzyme Q 10 dans les préparations cosmétiques ou dermatologiques obtenues est sélectionée dans le domaine allant de 0,0001 à 10,0 en pourcentage du poids, de préférence 0,01 -5,0 en pourcentage du poids par rapport au poids total des préparations.

3. Des préparations selon la revendication 1 **caractérisées par le fait que** la quantité totale de coenzyme Q 10 dans les préparations cosmétiques ou dermatologiques obtenues est sélectionée dans le domaine allant de 0,0001 à 20,0 en pourcentage du poids, de préférence 0,02 -10,0 en pourcentage du poids par rapport au poids total des préparations.

4. Des préparations selon la revendication 1 **caractérisées par le fait que** l'ajout moléculaires ou les ajouts moléculaires de cyclodextrine et de coenzyme Q 10 présentent les rapports molaires suivants :
1 molécule alpha cyclodextrine : 1 mol. coenzyme Q 10
1 molécule bêta cyclodextrine : 1 mol. coenzyme Q 10
1 molécule gamma cyclodextrine : 1 mol. coenzyme Q 10
2 molécule alpha cyclodextrine : 1 mol. coenzyme Q 10
2 molécule bêta cyclodextrine : 1 mol. coenzyme Q 10
2 molécule gamma cyclodextrine : 1 mol. coenzyme Q 10
1 molécule alpha cyclodextrine : 2 mol. coenzyme Q 10
1 molécule bêta cyclodextrine : 2 mol. coenzyme Q 10
1 molécule gamma cyclodextrine : 2 mol.coenzyme Q 10
3 molécule alpha cyclodextrine : 1 mol. coenzyme Q 10
3 molécule bêta cyclodextrine : 1 mol. coenzyme Q 10
3 molécule gamma cyclodextrine : 1 mol. coenzyme Q 10
